# EUROPÄISCHE PATENTSCHRIFT

(11) **EP 2 101 856 B1**
(45) Veröffentlichungstag und Bekanntmachung des Hinweises auf die Patenterteilung: **10.06.2015**
(21) Anmeldenummer: 07845274.5
(22) Anmeldetag: 30.11.2007
(51) Int. Cl.: A61M 25/10, A61M 1/10, A61B 17/12, A61M 1/36, A61M 25/00

(54) **VORRICHTUNG ZUR INTERMITTIERENDEN OKKLUSION EINER DAS ORGANSYSTEM DRAINIERENDEN VENE**
DEVICE FOR INTERMITTENT OCCLUSION OF A VEIN DRAINING THE ORGAN SYSTEM
DISPOSITIF POUR L'OCCLUSION INTERMITTENTE D'UNE VEINE DRAINANT LE SYSTÈME ORGANIQUE

(30) Priorität: 30.11.2006 AT 19992006
(43) Veröffentlichungstag der Anmeldung: 23.09.2009
(73) Patentinhaber: Miracor Medical Systems GmbH, 1060 Vienna (AT)
(72) Erfinder: Mohl, Werner, 2571 Altenmarkt/Thennenberg (AT)
(74) Vertreter: Keschmann, Marc
(86) Internationale Anmeldenummer: PCT/AT2007/000543
(87) Internationale Veröffentlichungsnummer: WO 2008/064387

(56) Entgegenhaltungen:
- EP-A- 0 230 996
- WO-A-03/008018
- DE-A1- 19 514 638

## Beschreibung

Die Erfindung bezieht sich auf eine Vorrichtung zur Durchführung eines Verfahrens zur intermittierenden Okklusion einer das Organsystem drainierenden Vene, bei welchem die Vene mit einer Okklusionseinrichtung okkludiert wird, der Flüssigkeitsdruck in der okkludierten Vene kontinuierlich gemessen und gespeichert wird, der Flüssigkeitsdruckverlauf als Funktion der Zeit ermittelt wird und die Okklusion der Vene in Abhängigkeit von wenigstens einer aus den Druckmesswerten abgeleiteten Kenngröße ausgelöst und/oder aufgehoben wird. Derartige Venen sind beispielsweise die iugularis interna oder der sinus coronarius.

Arterielles Blut, das den Herzmuskel versorgt, kann durch gesundes Herzgewebe hindurchtreten und es ernähren, hat jedoch Schwierigkeiten, das ischämische Gewebe zu erreichen. Dadurch wird die Versorgung des ischämischen Gewebes mit Nährstoffen sowie das Abführen von Abbauprodukten des Metabolismus von dem ischämischen Gewebe behindert.

In diesem Zusammenhang ist bereits vorgeschlagen worden, das ischämische Gewebe durch retrograde Perfusion mit Blut zu versorgen. Dabei wird versucht, das Blut in Gegenrichtung von dem Koronarsinus zurück durch das koronare Venensystem fließen zu lassen, in dem man aus einer anderen Quelle Blut in den Koronarsinus einspeist, entweder durch permanente Verbindung einer Arterie mit dem Koronarsinus oder durch temporäres Einsetzen eines Katheters in den Sinus, der mit von einer entfernten Arterie entnommenem und mit Hilfe einer sich außerhalb des Körpers des Patienten befindlichen Blutpumpe beförderten Blut versorgt wird.

Bei der eingangs vorgeschlagenen Technik für die Retroperfusion wird ein am Ende eines Katheters festgelegter aufblasbarer Ballon verwendet, um den Koronarsinus intermittierend zu okkludieren. Der Blutdruck im Koronarsinus steigt während der Okklusion bei jedem Herzschlag, sodass Blut, das durch das gesunde Gewebe des Herzmuskels in den Koronarsinus gelangt, in das ischämische Gewebe zurückgeschwemmt wird. Bei einer solchen intermittierenden Koronarsinusokklusion wird das Ballonende des Katheters perkutan oder durch eine Operation eingesetzt. Das andere Ende des Katheters wird mit Gas oder Flüssigkeit durch eine Pumpe versorgt, die ein zyklisches Aufblasen und Zusammenfallen des Ballons bewirkt.

Ein typisches Anwendungsgebiet für die Retroinfusion von Blut in Koronarvenen mittels intermittierender Koronarsinusokklusion betrifft die Myokardprotektion während eines kurzfristigen Koronararterienverschlusses im Rahmen eines kardiologischen Eingriffes. Ein typischer derartiger Eingriff ist beispielsweise die Ballondilatation einer arteriosklerotisch verengten Koronararterie. Bei dieser auch als perkutane transluminale Koronarangioplastie (PTCA) bekannten Methode wird ein Ballonkatheter unter Röntgenkontrolle in den Bereich der Stenose der Koronararterie geführt und die arteriosklerotische Plaque durch Aufblasen des am Ende des Katheters befindlichen Ballons komprimiert. Während der Dilatation des Ballons findet stromabwärts in der Arterie keine Versorgung des Gewebes mit sauerstoffhaltigem Blut statt, wobei sich bereits bei Dilatationen von länger als 30 Sekunden Dauer funktionelle Veränderungen im Ischämiegebiet des Myokards feststellen lassen. Entsprechende Probleme der Ischämieprotektion des Myokards stellen sich auch bei anderen Eingriffen zur Koronarvaskularisierung wie z.B. bei Atherektomie, Koronarendoprothesen, Laseranwendungen und perkutanen Herzklappenoperationen.

Eine Vorrichtung zur Retroinfusion von Koronarvenen ist beispielsweise aus der EP 230 996 A2 bekanntgeworden, mit welcher eine druckgesteuerte intermittierende Koronarsinusokklusion vorgenommen werden kann. Die Vorrichtung umfasst eine Einrichtung zum Okkludieren des Sinus wie z.B. einen aufblasbaren Ballonkatheter, eine Druckmesseinrichtung zum Messen des Flüssigkeitsdruckes innerhalb des Koronarsinus und ein Steuergerät, das Auslösesignale für die Okklusions-Einrichtung erzeugt, um eine Okklusion auszulösen oder aufzuheben. Das Steuergerät ist hiebei derart ausgelegt, dass im Koronarsinus das Druckmaximum während jedes Herzschlages gemessen, ein Plateauwert der Druckmaxima aufeinanderfolgender Herzschläge rechnerisch abgeschätzt und die Okklusion des Koronarsinus auf Basis des Plateauwertes der Druckmaxima aufgehoben wird.

Das Okkludieren des Koronarsinus bewirkt einen Druckanstieg und in der Folge eine Retroperfusion von Blut über die entsprechende Vene in die nutritiven Kapillaren des Ischämiegebietes, sodass diese Gebiete mit Nährstoffen versorgt werden können. Bei Aufheben der Okklusion wird das retroperfundierte Blut ausgeschwemmt, wobei gleichzeitig die Abfallprodukte des Metabolismus abgeführt werden.

In einer Reihe von Untersuchungen konnte gezeigt werde, dass endotheliale Wachstumsfaktoren unter anderem auf Anwendung von mechanischen Belastungen, und insbesondere von Druck, reagieren. Prinzipiell wirken auf das Endothel beim Durchströmen der Gefäße mit Blut neben Scherkräften naturgemäß auch die eingangs erwähnten Drücke, wobei eine möglichst lang andauernde Druckerhöhung prinzipiell vermehrt zur Freisetzung von gefäßbildenden Genen (VEGF-Genen, den vaskulären endothelialen Wachstumsfaktor kodierenden Genen) führt, wodurch die Regeneration der Herzgefäße und insbesondere die Angioneogenese begünstigt wird. Eine beliebig lang andauernde Druckerhöhung lässt sich aber durch Okklusion mit den bekannten Methoden nicht erzielen, da ja intermittierend nach Erreichen des Plateaus die Okklusion wieder aufgehoben werden muss und damit der Druck wieder abgebaut wird.

Die Erfindung zielt nun darauf ab, den auf eine Druckerhöhung bzw. Wechseldruckempfindlichkeit zurückzuführenden Beitrag zur Neoangiogenese zu erhöhen und diese theoretisch als positiv erkannten Effekte zu verbessern. Zur Lösung dieser Aufgabe kann bei der Erfindung nun ausgehend von dem eingangs vorbeschriebenen Verfahren während der Okklusion pulsierend Druck aufgebracht werden.

In Versuchen konnte gezeigt werden, dass ein linearer Druckanstieg ebenso wie die bloße Scherbelastung des Endothels wesentlich geringere Beiträge zur Produktion von vaskulären endothelialen Wachstumsfaktoren und damit wesentlich geringere Beiträge zur Neoangiogenese liefert. Erst durch die erfindungsgemäß vorgeschlagene pulsierende Druckbeaufschlagung wird dieser Effekt verbessert, wobei es in diesem Zusammenhang wesentlich ist, dass dieser pulsierende Druck während der Okklusion und damit in der Phase aufgebracht wird, in welcher generell ein Druckaufbau bis zum Erreichen eines Plateaus erfolgt. In besonders vorteilhafter Weise wird das erfindungsgemäße Verfahren hierbei so durchgeführt, dass der pulsierende Druckaufbau pneumatisch oder hydraulisch über eine schwingende, pulsierende Membran, insbesondere einen weiteren Ballon, oder piezoelektrisch aufgebracht wird, wobei vorzugsweise Druckwellen mit einer Frequenz zwischen 50 und 250 pro Minute, insbesondere 100 bis 200 pro Minute, eingebracht werden. Prinzipiell ist die Frequenz dieser Druckwellen bevorzugt höher zu wählen als dies dem Pulsschlag eines erwachsenen Menschen entsprechen würde. Die relativ hohen Pulsfrequenzen von 100 bis 200 pro Minute entsprechen weitestgehend Pulsfrequenzen, wie sie bei Neugeborenen und insbesondere in der Phase der Entwicklung des Herzens beobachtet werden. Derartige Frequenzen haben sich als besonders vorteilhaft für die Angio-neogenese herausgestellt. In besonders vorteilhafter Weise wird das Verfahren so durchgeführt, dass die Druckwellenmaxima mit EKG-Signalen, Herztönen und/oder phasischen Temperaturänderungen im Blutfluss synchronisiert werden.

Die erfindungsgemäße Vorrichtung zur intermittierenden Okklusion der Vene umfassend eine Okklusionseinrichtung, eine Druckmesseinrichtung zur kontinuierlichen Messung des Flüssigkeitsdrucks in der okkludierten Vene und einen Speicher für den Flüssigkeitsdruckverlauf als Funktion der Zeit ist hierbei im wesentlichen dadurch gekennzeichnet, dass Mittel vorgesehen sind zum Aufbringen von pulsierendem Druck in der okkludierten Vene. Außerdem ist eine die Ausbildung der Druckwellen steuernde Einrichtung vorgesehen, die so geschaltet ist, dass sie ab einem unteren Schwellwert des erfassbaren Druckanstiegs nach der Okklusion Druckwellen generiert und bei Aufhebung der Okklusion die Erzeugung von Druckwellen abschaltet. Eine solche Vorrichtung ist zur Ausführung des erfindungsgemäßen Verfahrens geeignet, wobei die Mittel zum Aufbringen des pulsierenden Drucks in aller Regel distal der Okklusionseinrichtung im okkludierten Bereich der Vene angeordnet sind.

In bevorzugter Weise sind die Mittel zum Aufbringen von pulsierendem Druck von einem Schwingkörper gebildet, wobei der Schwingkörper zur Ausbildung von Druckwellen antreibbar ist. Ein solcher Schwingkörper kann die Ausbildung von Druckwellen nach unterschiedlichen Prinzipien hervorrufen, wobei der Schwingkörper in bevorzugter Weise in einem nach Art eines Stents ausgebildeten Käfig angeordnet ist. Der im Rahmen dieser Vorrichtung bevorzugt vorgesehene Käfig sichert den Bereich, in welchem der Schwingkörper zur Wirkung gelangen soll, nach Art eines Stents ab und verhindert eine unmittelbare Kollision des Schwingkörpers mit der Gefäßwand. Auf diese Weise können die gewünschten pulsierenden Druckwellen effizient und mit geringem mechanischem Aufwand aufgebaut werden, wobei der Druckaufbau prinzipiell durch verschiedene mechanische Wandler erzielt werden kann. Besonders bevorzugt ist hierbei eine Ausbildung, bei welcher der Schwingkörper von einem pneumatisch oder hydraulisch aufweitbaren Hohlkörper bzw. Ballon gebildet ist.

Um sicherzustellen, dass die höherfrequenten Pulsationen auch entsprechend höhere Druckspitzenwerte ergeben, und damit die Stimulation des Endothels in der gewünschten Weise erfolgt, ist die Ausbildung mit Vorteil so getroffen, dass der Schwingkörper mit der Okklusionseinrichtung gekoppelt ist und in der Zeit des okklusionsbedingten Druckaufbaus zusätzlich zu Pulsationen antreibbar ist.

Prinzipiell wird bei der eingangs genannten Vorrichtung eine Druckmesseinrichtung so eingesetzt, dass nach Abschätzung des jeweiligen Plateauwertes, wie er in der Folge einer Okklusion auftritt, auch eine entsprechende Entlastung des Ballons erfolgt und die Okklusion wieder aufgehoben wird. Dieser Messwert ist aber für den tatsächlichen Erfolg des pulsierenden Drucks nicht relevant, sodass eine entsprechende Kontrolle nicht ohne weiteres möglich ist. Mit Vorteil ist die Ausbildung daher so getroffen, dass die Druckmesseinrichtung zur Messung des Flüssigkeitsdrucks im okkludierten Koronarsinus sowie zur Messung der durch den Schwingkörper eingebrachten Druckspitzen ausgebildet ist.

In bevorzugter Weise ist die Ausbildung so getroffen, dass die Mittel zum Aufbringen von pulsierendem Druck von einer Vorrichtung zum pulsierenden Einbringen von Perfusat in die okkludierte Vene gebildet sind. Mit einer solchen Ausbildung können nicht nur die gewünschten Druckwellen hervorgerufen werden, sondern es ist zusätzlich möglich, beispielsweise frisches Blut zur Ernährung des ischämischen Gewebes einzusetzen, wobei auch Blut ersetzt werden kann, welches an anderer Stelle im Zuge des Eingriffes dem Blutkreislauf verloren gegangen ist.

Zur Synchronisierung der Druckwellenmaxima mit relevanten Parametern für die Funktion des Kreislaufs ist die Ausbildung bevorzugt so getroffen, dass eine die Ausbildung der Druckwellen steuernde Einrichtung mit Signalleitungen für EKG-Signale, Herztöne und/oder Temperaturmesswerte verbindbar ist.

Mit Vorteil ist die erfindungsgemäße Vorrichtung dahingehend weitergebildet, dass die Ausbildung der Druckwellen in Abhängigkeit von den Blutdruckspitzen, welche vom Herzen produziert werden, hervorrufbar ist. Auf diese Weise kann die Ausbildung der Druckwellen mit dem Auftreten der Blutdruckspitzen abgestimmt werden, sodass die retrograde Perfusion des ischämischen Gewebes mit geringem Aufwand besonders effektiv gelingt.

In bevorzugter Weise kann die Ausbildung der Druckwellen durch eine im Lumen pulsierend geförderte Flüssigkeit hervorgerufen werden. Eine solche pneumatisch angetriebene Ausbildung zeichnet sich durch eine einfache Handhabung sowie durch ein besonders direktes Ansprechverhalten aus. Die Ausbildung der Druckwellen kann darüber hinaus in bevorzugter Weise durch Schallwellen und insbesondere durch Ultraschall sowie, wie es einer bevorzugten Ausführungsform der erfindungsgemäßen Vorrichtung eintspricht, durch elektromechanische und insbesondere durch elektromechanische Wellen hervorgerufen werden. Auf diese Wiese können Druckwellen von praktisch beliebiger Frequenz ausgebildet werden.

Gemäß einer bevorzugten Ausbildungsform ist die erfindungsgemäße Vorrichtung dahingehend weitergebildet, dass die Ausbildung der Druckwellen durch eine Kombination der Wirkung der Okklusionseinrichtung und der Wirkung der Mittel zum Aufbringen von pulsierendem Druck in der Vene hervorrufbar ist. Auf diese Weise kann zusätzlich zum Mittel zum Aufbringen von pulsierendem Druck die Okklusionseinrichtung zur Erzeugung von Druckwellen genutzt werden, sodass besonders kräftige Druckwellen hervorrufbar sind.

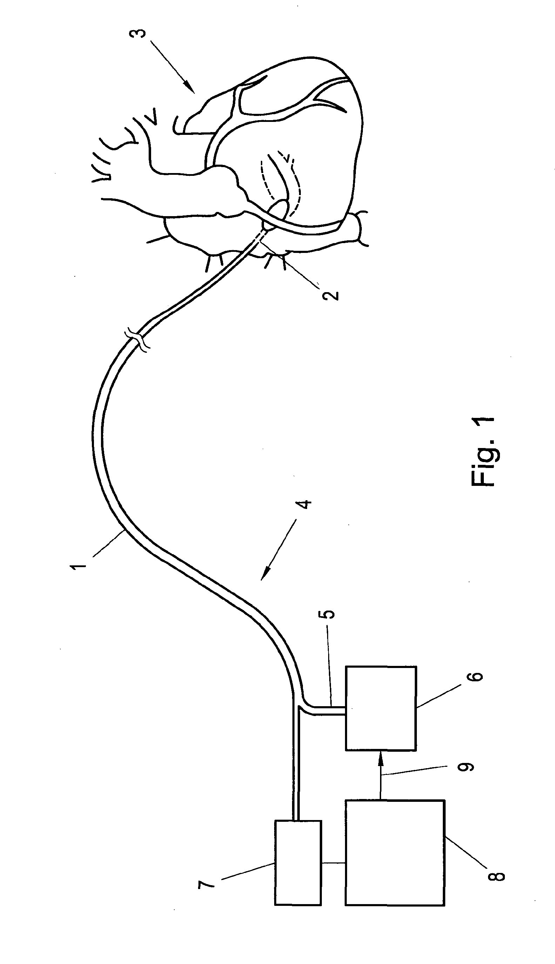

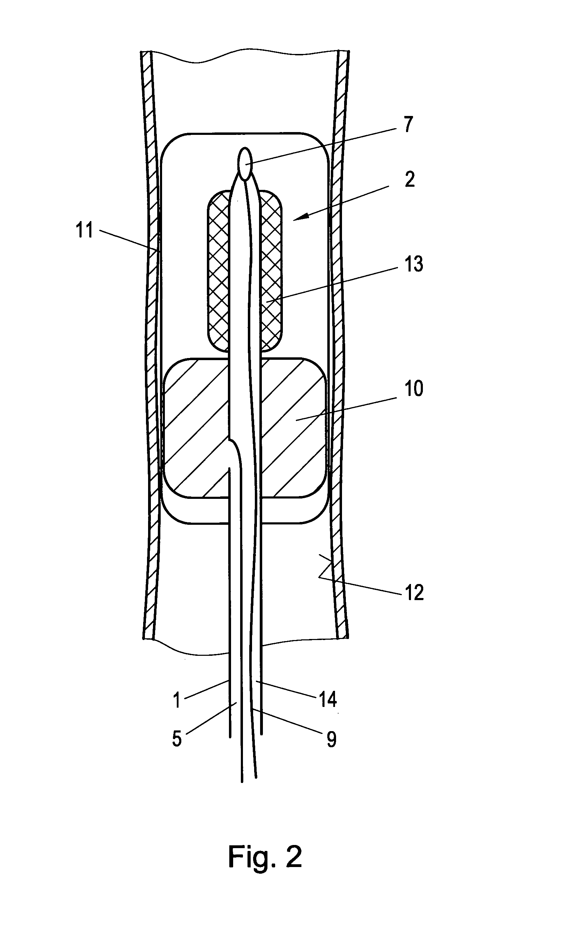

Die Erfindung wird nachfolgend anhand eines in der Zeichnung dargestellten Ausführungsbeispiels näher erläutert. In dieser zeigen Fig.1 eine Diagrammansicht eines Herzens mit einer Vorrichtung zum intermittierenden Okkludieren des Koronarsinus und Fig.2 eine vergrößerte Darstellung des distalen Endes des erfindungsgemäßen Katheters.

In Fig. 1 ist schematisch die Vorrichtung zum intermittierenden Okkludieren des Koronarsinus dargestellt, wobei ein Multilumen-Katheter 1, dessen distales Ende 2 in den Koronarsinus des Herzens 3 über das Atrium eingesetzt wird, ersichtlich ist. Das proximale Ende 4 des Katheters 1 hat ein Ballonaufblaslumen 5, das mit einer Pumpe 6 verbunden ist. Der am distalen Ende 2 des Katheters 1 herrschende Druck wird von einer Druckmesseinrichtung 7 erfasst, wobei die Druckmesseinrichtung auch einen Speicher für die ermittelten Messwerte aufweist. Die jeweiligen Druckmesswerte werden einer Steuerungseinrichtung 8 umfassend eine Recheneinheit zugeführt, welche Steuersignale über die Leitung 9 zum Starten und Anhalten der Pumpe 6 liefert.

In Fig.2 ist das distale Ende 2 des Multilumen-Katheters 1 vergrößert dargestellt. Das Ballonaufblaslumen mündet hier in den in Fig.2 in aufgeweiteter und damit okkludierender Position eingezeichneten Ballon 10. Das distale Ende wird von einem stentartigen Käfig 11, welcher an die Gefäßwand 12 anpressbar ist, gesichert, sodass eine unmittelbare Kollision dieses Endes mit der Aorten- bzw. Gefäßwand vermieden wird. Das distale Ende trägt neben dem Ballon 10 nun auch noch einen Vibratorkörper 13, der beispielsweise über das Lumen 14 mit hydraulischen Druckpulsen beaufschlagt werden kann. Während der Okklusion, d.h. bei aufgeblasenem Ballon 10 wird somit über das Lumen 14 Druckmedium pulsierend in den Vibratorkörper 13 geleitet, sodass die entsprechende Druckwechselbeanspruchung mit der auf diese Weise verbesserten Stimulation der eine Neoangiogenese auslösenden Faktoren erzielt wird.

## Patentansprüche

1. Vorrichtung zur intermittierenden Okklusion einer Vene umfassend eine Okklusionseinrichtung, eine Druckmezseinrichtung zur kontinuierlichen Messung des Flüssigkeitsdrucks in der okkludierten Vene und einen Speicher für den Flüssigkeitsdruckverlauf als Funktion der Zeit, **dadurch gekennzeichnet, dass** Mittel vorgesehen sind zum Aufbringen von pulsierendem Druck in der okkludierten Vene in der Phase, in welcher generell ein Druckaufbau erfolgt, nämlich Druckwellen mit einer Frequenz zwischen 50 und 250 pro Minute, insbesondere 100 bis 200 pro Minute, und dass eine die Ausbildung der Druckwellen steuernde Einrichtung (8) vorgesehen ist, die so geschaltet ist, dass sie ab einem unteren Schwellwert des erfassbaren Druckanstiegs nach der Okklusion Druckwellen generiert und bei Aufhebung der Okklusion die Erzeugung von Druckwellen abschaltet.

2. Vorrichtung nach Anspruch 1, dadurch gekenntzeichnet, dass die Mittel zum Aufbringen von pulsierendem Druck von einem Schwingkörper (13) gebildet sind, wobei der Schwingkörper (13) zur Ausbildung von Druckwellen antreibbar ist.

3. Vorrichtung nach Anspruch 1 oder 2, **dadurch gekennzeichnet, dass** der Schwingkörper (13) in einem nach Art eines Stents ausgebildeten Käfig (11) angeordnet ist.

4. Vorrichtung nach Anspruch 1, 2 oder 3, **dadurch gekennzeichnet, dass** der Schwingkörper (13) von einem pneumatisch oder hydraulisch aufweitbaren Hohlkörper bzw. Ballon (10) gebildet ist.

5. Vorrichtung nach einem der Ansprüche 1 bis 4, **dadurch gekennzeichnet, dass** der Schwingkörper (13) mit der Okklusionseinrichtung gekoppelt ist und in der Zeit des okklusionsbedingten Druckaufbaus zusätzlich zu Pulsationen antreibbar ist.

6. Vorrichtung nach einem der Ansprüche 1 bis 5, **dadurch gekennzeichnet, dass** die Druckmesseinrichtung (7) zur Messung des Flüssigkeitsdruckes im okkludierten Koronarsinus sowie zur Messung der durch den Schwingkörper (13) eingebrachten Druckspitzen ausgebildet ist.

7. Vorrichtung nach Anspruch 1, **dadurch gekennzeichnet, dass** die Mittel zum Aufbringen von pulsierendem Druck von einer Vorrichtung zum pulsierenden Einbringen von Perfusat in die okkludierte Vene gebildet sind.

8. Vorrichtung nach einem der Ansprüche 1 bis 7, **dadurch gekennzeichnet, dass** eine die Ausbildung der Druckwellen steuernde Einrichtung (8) vorgesehen ist, die mit Signalleitungen für EKG-Signale, Herztöne und/oder Temperaturmesswerte verbindbar ist.

9. Vorrichtung nach einem der Ansprüche 1 bis 8, **dadurch gekennzeichnet, dass** die Ausbildung der Druckwellen in Abhängigkeit von den Blutdruckruckspitzen, welche vom Herzen (3) produziert werden, hervorrufbar ist.

10. Vorrichtung nach einem der Ansprüche 1 bis 9, **dadurch gekennzeichnet, dass** die Ausbildung der Druckwellen durch eine im Lumen (14) pulsierend geförderte Flüssigkeit hervorrufbar ist.

11. Vorrichtung nach einem der Ansprüche 1 bis 10, **dadurch gekennzeichnet, dass** die Ausbildung der Druckwellen durch Schallwellen und insbesondere durch Ultraschall hervorrufbar ist.

12. Vorrichtung nach einem der Ansprüche 1 bis 11, **dadurch gekennzeichnet, dass** die Ausbildung der Druckwellen durch elektromechanische und insbesondere durch nanoelektromechanische Wellen hervorrufbar ist.

13. Vorrichtung nach einem der Ansprüche 1 bis 12, **dadurch gekennzeichnet, dass** die Ausbildung der Druckwellen durch eine Kombination der Wirkung der Okklusionseinrichtung und der Wirkung der Mittel zum Aufbringen von pulsierendem Druck in der Vene hervorrufbar ist.

## Claims

1. A device for the intermittent occlusion of a vein, including an occlusion device, a pressure measuring device for continuously measuring the fluid pressure in the occluded vein, and a memory for storing the fluid pressure behaviour as a function of time, **characterized in that** means are provided for applying a pulsating pressure in the occluded vein **in that** phase, in which a pressure build-up generally occurs, namely pressure waves at frequencies of between 50 and 250 per minute and, in particular, 100 to 200 per minute and that a device (8) controlling the formation of said pressure waves is arranged to generate pressure waves from a lower threshold value of the detectable pressure increase after the occlusion and to stop the generation of pressure waves upon release of the occlusion.

2. A device according to claim 1, **characterized in that** the means for applying a pulsating pressure are comprised of an oscillating body (13), said oscillating body (13) being drivable to form pressure waves.

3. A device according to claim 1 or 2, **characterized in that** said oscillating body (13) is arranged in a cage (11) designed in the manner of a stent.

4. A device according to claim 1, 2 or 3, **characterized in that** the oscillating body (13) is comprised of a pneumatically or hydraulically expandable hollow body or balloon (10).

5. A device according to any one of claims 1 to 4, **characterized in that** the oscillating body (13) is coupled with the occlusion device and additionally drivable to pulsations during the time of the occlusion-related pressure build-up.

6. A device according to any one of claims 1 to 5, **characterized in that** the pressure measuring device (7) is designed to measure the fluid pressure in the occluded coronary sinus and to measure the pressure peaks introduced by the oscillating body (13).

7. A device according to claim 1, **characterized in that** the means for applying a pulsating pressure is comprised of a device for the pulsating introduction of perfusate intro the occluded vein.

8. A device according to any one of claims 1 to 7, **characterized in that** a device (8) controlling the formation of said pressure waves is connectable with signal lines for ECG signals, heart tones and/or temperature measurements.

9. A device according to any one of claims 1 to 8, **characterized in that** the formation of said pressure waves is inducible as a function of the blood pressure peaks produced by the heart (3).

10. A device according to any one of claims 1 to 9, **characterized in that** the formation of said pressure waves is inducible by a fluid pulsingly conveyed in the lumen (14).

11. A device according to any one of claims 1 to 10, **characterized in that** the formation of said pressure waves is inducible by sonic waves and, in particular, ultrasonic waves.

12. A device according to any one of claims 1 to 11, **characterized in that** the formation of said pressure waves is inducible by electromechanical and, in particular, nanoelectromechanical waves.

13. A device according to any one of claims 1 to 12, **characterized in that** the formation of said pressure waves is inducible by a combination of the action of the occlusion device and the action of the means for applying a pulsating pressure in the vein.

## Revendications

1. Dispositif d'occlusion intermittente d'une veine, comprenant un système d'occlusion, un système de mesure de pression pour la mesure continue de la pression de fluide dans la veine occluse et une mémoire de l'évolution de pression de fluide en function du temps, **caractérisé en ce que** des moyens sont prévus pour l'application d'une pression pulsée dans la veine occluse dans la phase dans laquelle une élévation de pression se produit généralement, à savoir des ondes de pression à une fréquence entre 50 et 250 par minute, en particulier de 100 à 200 par minute, et **en ce que**
un système (8) commandant la formation des ondes de pression est prévu, lequel est enclenché de telle sorte que, à partir d'une valeur seuil inférieure de l'augmentation de pression captable après l'occlusion, des ondes de pression sont générées et, lors de la surpression de l'occlusion, la génération d'ondes de pression est arrêtée.

2. Dispositif selon la revendication 1, **caractérisé en ce que** les moyens d'application d'une pression pulsée sont formés d'un corps oscillant (13), le corps oscillant (13) pouvant être actionné pour la constitution d'ondes de pression.

3. Dispositif selon la revendication 1 ou 2, **caractérisé en ce que** le corps oscillant (13) est disposé dans une cage (11) constituée à la manière d'un stent.

4. Dispositif selon la revendication 1, 2 ou 3, **caractérisé en ce que** le corps oscillant (13) est formé d'un corps creux ou d'un ballonnet (10) pouvant être dilaté de manière pneumatique ou hydraulique.

5. Dispositif selon l'une des revendications 1 à 4, **caractérisé en ce que** le corps oscillant (13) est couplé avec le système d'occlusion et peut en outre être actionné en pulsations dans la période d'élévation de pression due à l'occlusion.

6. Dispositif selon l'une des revendications 1 à 5, **caractérisé en ce que** le système de mesure de pression (7) est constitué pour la mesure de la pression de fluide dans le sinus coronaire occlus et pour la mesure de pointes de pressions introduites par le corps oscillant (13).

7. Dispositif selon la revendication 1, **caractérisé en ce que** les moyens d'application d'une pression pulsée par un dispositif pour l'introduction pulsée de perfusat sont formés dans la veine occluse.

8. Dispositif selon l'une des revendications 1 à 7, **caractérisé en ce qu'**un système (8) commandant la constitution des ondes de pression est prévu, lequel peut être relié avec des lignes de signal pour des signaux d'ECG, des bruits cardiaques et/ou des valeurs de mesure de température.

9. Dispositif selon l'une des revendications 1 à 8, **caractérisé en ce que** la constitution des ondes de pression peut être déclenchée en fonction des pointes de pression de tension artérielle qui sont produites par le coeur (3).

10. Dispositif selon l'une des revendications 1 à 9, **caractérisé en ce que** la constitution des ondes de pression peut être déclenchée par un fluide acheminé de façon pulsée dans le lumen (14).

11. Dispositif selon l'une des revendications 1 à 10, **caractérisé en ce que** la constitution des ondes de pression peut être déclenchée par des ondes sonores et en particulier par ultrasons.

12. Dispositif selon l'une des revendications 1 à 11, **caractérisé en ce que** la constitution des ondes de pression peut être déclenchée par des ondes électromécaniques et en particulier par des ondes nano-électromécaniques.

13. Dispositif selon l'une des revendications 1 à 12, **caractérisé en ce que** la constitution des ondes de pression peut être déclenchée par une combinaison de l'action du système d'occlusion et de l'action des moyens pour l'application d'une pression pulsée dans la veine.
